# EUROPEAN PATENT APPLICATION

(11) **EP 4 803 594 A1**
(43) Date of publication of application: **09.09.2026**
(21) Application number: 24907654.8
(22) Date of filing: 29.07.2024
(51) Int. Cl.: C10G 2/00, C10J 3/72, C01B 3/38, C01B 3/48, C01B 32/40, C07C 1/20, C07C 29/151, C07C 31/04

(54) **METHOD AND APPARATUS FOR PRODUCING HYDROCARBON**

(30) Priority: 18.12.2023 KR 20230184281
(71) Applicant: SK Innovation Co., Ltd., Seoul 03188 (KR)
(72) Inventor: JEON, Heejung, Daejeon 34124 (KR); KIM, Okyoun, Daejeon 34124 (KR); YUN, Dongmin, Daejeon 34124 (KR)
(74) Representative: Lorenz Seidler Gossel Part. mbB
(86) International application number: PCT/KR2024/011005
(87) International publication number: WO 2025/135367

(57) **Abstract**

The present disclosure provides a method for producing hydrocarbon, comprising the steps of: (S1) generating a first mixed gas by heat-treating organic waste; (S2) generating a second mixed gas by steam-reforming the first mixed gas in a first fixed-bed reactor; (S3) separating the second mixed gas into a first stream including carbon dioxide and a second stream including hydrogen and carbon monoxide; (S4) introducing the first stream obtained by separation in step (S3) into a second fixed-bed reactor and converting the first stream into carbon monoxide through a reverse Boudouard reaction; (S5) producing a third mixed gas by mixing the second stream and the carbon monoxide obtained by conversion in step (S4); (S6) generating a synthetic gas from the third mixed gas through a water gas shift reaction; and (S7) producing hydrocarbon from the synthetic gas through a catalytic reaction.

## Description

### [Technical Field]

The present disclosure relates to a method or an apparatus for producing a hydrocarbon from organic wastes, and more particularly, to a method and an apparatus for producing a hydrocarbon, which may improve a production yield of a hydrocarbon from organic wastes and minimize carbon dioxide production.

### [Background Art]

Organic wastes may seriously damage the environment by decay when landfilled, and should be discarded through a prescribed treatment process after collecting them according to their properties when discarded. However, since simple disposal of the organic wastes requires securing treatment facilities and consuming a large amount of manpower and is more wasteful than productive, methods and technologies for recycling organic wastes have been developed in recent years. Representatively, a gasification process technology in which synthetic gas is produced using organic wastes and converted into a high value-added product for energization may be mentioned.

The gasification process generally refers to a series of processes of reacting carbonaceous raw materials such as coal, organic wastes, and biomass under the supply of water vapor, oxygen, carbon dioxide, or a mixture thereof to convert the raw materials into synthetic gas including hydrogen and carbon monoxide, in which the "synthetic gas" refers to a mixed gas which is usually produced by a gasification reaction and includes hydrogen and carbon monoxide and may further include carbon dioxide and/or methane.

The gasification process technology has expanded to a technology of producing fuels and raw materials of various compounds, and for example, the synthetic gas may be used as the raw material of a Fischer-Tropsch synthesis reaction to manufacture high value-added products such as light oil, heavy oil, diesel oil, wax, jet oil, and lubricant base oil. Besides, it is known that the technology may be applied to hydrogen power generation, ammonia manufacture, an oil refining process, and the like, using hydrogen in the synthetic gas which is the main product of the gasification process, and high value-added chemicals such as acetic acid, olefin, dimethyl ether, aldehyde, fuel, and an additive may be obtained, using methanol manufactured from the synthetic gas. However, since the synthetic gas produced from organic wastes has a very poor production yield, it is difficult to effectively produce a high value-added compound material from the synthetic gas.

Recently, as a process for manufacturing synthetic gas, a gasification process using a catalyst has been carried out, but due to the formation of coke and the like in the gasification process, the catalyst is deactivated causing process trouble by the deactivated catalyst during a continuous operation. In addition, for securing economic feasibility, relatively expensive catalysts need to be recovered, but in order to recover catalyst discharged in the state in which coke and the like are agglomerated, a plurality of subsequent processes (such as air burning) should be carried out, and thus, process efficiency is significantly deteriorated.

In addition, since the conventionally performed gasification process of organic wastes has a significantly low synthetic gas production yield of 30% or less, the synthetic gas being converted into a high value-added product, and thus, has poor productivity, there are limitations to utilizing or commercializing the process. Further, in terms of environmental protection, it is preferred to suppress CO₂ emission, but since the gasification reaction product of organic wastes contains CO₂ in addition to H₂ and CO, more carbon dioxide is emitted as compared with the case of landfill or pyrolysis treatment, and thus, the gasification process has a serious problem of causing rather increased environmental pollution.

Thus, there is a need for a method and an apparatus for producing a hydrocarbon, which may improve a production yield of synthetic gas which may be converted into a high value-added product during a gasification process of organic wastes and efficiently convert the synthetic gas into a high value-added hydrocarbon, while minimizing carbon dioxide emissions.

### [Disclosure]

### [Technical Problem]

An object of the present disclosure is to provide a method and an apparatus for producing a hydrocarbon, which have a significantly improved production yield of hydrocarbon.

Another object of the present disclosure is to provide a method and an apparatus for producing a hydrocarbon, which may minimize carbon dioxide emissions.

### [Technical Solution]

In one general aspect, a method for producing a hydrocarbon includes: (S1) heat-treating organic wastes to produce a first mixed gas; (S2) steam-reforming the first mixed gas in a first fixed-bed reactor to produce a second mixed gas; (S3) separating the second mixed gas into a first stream including carbon dioxide and a second stream including hydrogen and carbon monoxide; (S4) introducing the first stream separated in (S3) into a second fixed-bed reactor and converting the first stream into carbon monoxide through a reverse Boudouard reaction; (S5) mixing the second stream and the carbon monoxide converted in (S4) to produce a third mixed gas; (S6) producing a synthetic gas from the third mixed gas through a water gas shift reaction; and (S7) producing a hydrocarbon from the synthetic gas through a catalytic reaction.

In an example, the first mixed gas may further include one or more selected from the group consisting of landfill gas, shale gas, refinery exhaust gas, and biogas.

In an example, the second fixed-bed reactor may be supplied with a carbon source from outside.

In an example, the first stream may include 50 vol% or more of carbon dioxide.

In an example, the pyrolysis gas may have a C/O element ratio of 0.1 or less.

In an example, (S2) may be performed under a composite catalyst in which an active metal is supported on a support.

In an example, the active metal may include one or more selected from the group consisting of nickel, vanadium, iron, platinum, palladium, and ruthenium.

In an example, the support may include one or more selected from the group consisting of silica, alumina, silica-alumina, carbon, zirconia, titania, zeolite, SAPO, and ALPO.

In an example, (S2) may be performed at a temperature of 700 to 1000°C.

In an example, (S4) may be performed at a temperature of 600 to 1000°C and a pressure of 50 to 300 KPa.

In an example, the synthetic gas in (S6) may include hydrogen and carbon monoxide, and a ratio between hydrogen and carbon monoxide may satisfy 1.8:1 to 2.2:1.

In an example, the organic wastes in (S1) may be one or more selected from the group consisting of waste plastic, solid waste, biomass, waste oil, waste tire, and standard plastic garbage bags.

In an example, a step of refining the first mixed gas of (S1) may be further included before (S2).

In another general aspect, an apparatus for producing a hydrocarbon includes: a pyrolysis reactor which heat-treats organic wastes to produce a first mixed gas; a fixed-bed reforming reactor which steam-reforms the first mixed gas under a catalyst to produce a second mixed gas; a carbon dioxide separation unit which separates the second mixed gas into a first stream including carbon dioxide and a second stream including hydrogen and carbon monoxide; a second fixed-bed reactor which converts the first stream into carbon monoxide through a reverse Boudouard reaction; a gas mixing unit which mixes the second stream and carbon monoxide converted from the second fixed-bed reactor to produce a third mixed gas; a synthetic gas production unit which converts the third mixed gas into a synthetic gas through a water gas shift reaction; and a hydrocarbon conversion unit which converts the synthetic gas into a hydrocarbon through a catalytic reaction.

In an example, the second fixed-bed reactor may be connected between the carbon dioxide separation unit and the gas mixing unit, and the second fixed-bed reactor may include a fixed-bed reactor 2-1 and a fixed-bed reactor 2-2 which are connected in parallel.

In an example, the second fixed-bed reactor may further include a control part which switches the fixed-bed reactor 2-1 and the fixed-bed reactor 2-2, and the control part may control performance of a first mode in which the fixed-bed reactor 2-1 is regenerated, and the reverse Boudouard reaction is performed by the fixed-bed reactor 2-2, by blocking a connection between the carbon dioxide separation unit - the fixed-bed reactor 2-1 - the gas mixing unit; or a second mode in which the fixed-bed reactor 2-2 is regenerated, and the reverse Boudouard reaction is performed by the fixed-bed reactor 2-1, by blocking a connection between the carbon dioxide separation unit - the fixed-bed reactor 2-2 - the gas mixing unit.

In an example, a purification unit may be further included between the pyrolysis reactor and the first fixed-bed reactor.

### [Advantageous Effects]

According to an example of the present disclosure, a production yield of hydrocarbon produced from organic wastes may be significantly improved.

According to an example of the present disclosure, hydrocarbon conversion may be performed using a process and an apparatus appropriate for a C/O element ratio of a feed, thereby significantly improving a production yield of hydrocarbon.

According to an example of the present disclosure, carbon dioxide emissions may be minimized in a production process of a hydrocarbon.

### [Description of Drawings]

FIG. 1 is a schematic diagram showing an apparatus for producing a hydrocarbon according to an example of the present disclosure.
FIG. 2 is a schematic diagram showing an apparatus for producing a hydrocarbon including a purification device according to an example.
FIG. 3 is a schematic diagram showing an apparatus for producing a hydrocarbon including a reverse Boudouard reactor capable of switching according to an example.

### [Best Mode]

The singular form of the term used herein may be intended to also include a plural form, unless otherwise indicated.

The numerical range used in the present specification includes all values within the range including the lower limit and the upper limit, all double limited values, and all possible combinations of the upper limits and the lower limits in the numerical range defined in different forms. Unless otherwise defined in the present specification, values which may be outside a numerical range due to experimental error or rounding off of a value are also included in the defined numerical range.

The term "comprise" mentioned in the present specification is an open-ended description having a meaning equivalent to the term such as "is/are provided", "contain", "have", or "is/are characterized", and does not exclude elements, materials, or processes which are not further listed.

The unit of % used in the present specification without particular mention refers to % by weight, unless otherwise defined.

As a conventional process for manufacturing synthetic gas, a gasification process using a catalyst has been carried out, but due to the formation of coke and the like in the gasification process, the catalyst is deactivated causing process trouble by the deactivated catalyst during a continuous operation. In addition, for securing economic feasibility, relatively expensive catalysts need to be recovered, but in order to recover catalyst discharged in the state in which coke is agglomerated, a plurality of subsequent processes (such as air burning) should be carried out, which significantly deteriorates process efficiency, and a large amount of carbon dioxide is emitted during the process causing environmental pollution. In addition, since the conventionally performed gasification process of organic wastes has a significantly low production yield of synthetic gas of 30% or less and poor productivity, there were limits to conversion of the synthetic gas into a high value-added product. Thus, the inventors of the present disclosure devised a production method and production apparatus which may efficiently produce hydrocarbons from organic wastes while minimizing carbon dioxide emissions.

The present disclosure provides a method for producing a hydrocarbon including: (S1) heat-treating organic wastes to produce a first mixed gas; (S2) steam-reforming the first mixed gas in a first fixed-bed reactor to produce a second mixed gas; (S3) separating the second mixed gas into a first stream including carbon dioxide and a second stream including hydrogen and carbon monoxide; (S4) introducing the first stream separated in (S3) into a second fixed-bed reactor and converting the first stream into carbon monoxide through a reverse Boudouard reaction; (S5) mixing the second stream and the carbon monoxide converted in (S4) to produce a third mixed gas; (S6) producing a synthetic gas from the third mixed gas through a water gas shift reaction; and (S7) producing a hydrocarbon from the synthetic gas through a catalytic reaction.

Since the method for producing a hydrocarbon according to the present disclosure may convert organic wastes into a synthetic gas with high efficiency as compared with a conventional gasification process, a yield of a high value-added hydrocarbon converted from the synthetic gas may be maximized. Specifically, in reforming gas derived from organic wastes, reforming efficiency is improved using a fixed-bed reactor having the highest conversion rate per catalyst weight, thereby improving the production yield of synthetic gas and hydrocarbon. In addition, carbon dioxide emissions may be minimized in a hydrocarbon production process to prevent environmental pollution.

(S1) is a step of heat treating organic wastes to produce a first mixed gas, in which a gasification reaction of the organic wastes may occur.

In an example, the organic wastes in (S1) may be one or more selected from the group consisting of waste plastic, solid waste, biomass, waste oil, waste tire, and standard plastic garbage bags.

Specifically, in (S1), any one or more gasification reactions selected from the following Reaction Formulae 1 to 4 may be involved:

[Reaction Formula 1] CₓH_{y} + H₂O → H₂ + CO (water gasification reaction)

[Reaction Formula 2] CₓH_{y} + CO₂ → CO (carbon dioxide gasification reaction)

[Reaction Formula 3] CO + 3H₂ → CH₄ + H2O (methanation reaction)

[Reaction Formula 4] CₓH_{y} + O₂→ CO₂ (oxidation reaction)

In an example, the first mixed gas may include methane, hydrogen, carbon monoxide, and carbon dioxide, and may include various impurities such as nitrogen oxides, sulfur oxides, and hydrogen chloride.

In an example, (S1) may be performed in a first catalyst condition or a non-catalytic condition in order to increase a methane content in the composition of the first mixed gas. Since it may be advantageous in terms of methane reforming reaction efficiency or synthetic gas production yield to increase a methane content in the composition of the first mixed gas, in order to increase the methane content in (S1), an acid site catalyst or molybdenum series forming catalyst may be used as a bed material of a gasifier. When the catalyst is not used, the operation may be performed with an increased methane content by operating the gasifier under the high temperature and high pressure conditions. The yield improvement effect from methane reforming may be expected identically in a C2 to C4 hydrocarbon gas content as well as the methane content, and the C2 to C4 gas content may also be increased by using the acid site catalyst or through low temperature and high pressure gasification operation.

In an example, the first catalyst may be an acid site catalyst or molybdenum-based forming catalyst. The acid site catalyst may be alumina or a catalyst having a solid acid site derived from a structure. The alumina may be alumina alone, silica-alumina, alumina dispersed in a carbon structure, and the like, and the structure acid site material may be zeolite, SAPO, AlPO, MOF, a structural variant thereof, and the like. The molybdenum-based catalyst refers to a catalyst in which molybdenum is supported on a support, and if necessary, nickel, cobalt, and the like may be added, or tungsten may be used instead of molybdenum. Any support may be used as long as it has durability to support an active metal, and for example, it may include materials including one or more selected from silica, alumina, silica-alumina, carbon, and zirconia.

As another example of increasing the methane content in the first mixed gas, the first mixed gas may further include one or more selected from the group consisting of landfill gas, shale gas, refinery exhaust gas, and biogas. Since the landfill gas, shale gas, refinery exhaust gas, and biogas include 40 vol% or more, specifically 50 vol% or more of methane and carbon dioxide, the first mixed gas further includes the gas described above, and thus, the production yield of synthetic gas may be further improved through a methane reforming reaction and a reverse Boudouard reaction.

In an example, a C/O element ratio of the first mixed gas may be 0.8 or less, 0.75 or less, or 0.7 or less as the upper limit and 0.1 or more, 0.2 or more, or 0.3 or more ad the lower limit. Specifically, it may be 0.1 to 0.8, more specifically 0.3 to 0.7.

When the C/O element ratio of the first mixed gas satisfies the range described above, an oxygen content is higher than a carbon content in the first mixed gas when a methane reforming reaction described later is performed, and thus, an amount of coke produced by a side reaction is significantly decreased to prevent deactivation of the catalyst, thereby performing the methane reforming reaction with high efficiency.

In an example, a step of purifying the first mixed gas may be further included, after (S1).

The first mixed gas produced by heat-treating organic wastes may include one or two or more impurities selected from the group consisting of tar, sulfur, nitrogen, and chlorine. Specifically, the first mixed gas may include water-soluble impurities such as H₂S, HCl, HOCl, and NH₃ and water-insoluble impurities such as tar. Since these impurities included in the first mixed gas may induce deactivation of the catalyst to reduce the efficiency of a subsequent process, when purification is performed after removing impurities from the first mixed gas, the efficiency of the entire process may be improved.

A method for purifying the first mixed gas may be one or a combination of two or more selected from the group consisting of use of a dust collection filter for high pressure, water washing, basic solution washing, passing through a ceramic filter, and the like, but is not limited thereto. When the water washing or basic solution washing is used, water-soluble impurities such as H₂S, HCl, HOCl, and NH₃ included in the first mixed may be removed, and when the first mixed gas is passed through a ceramic filter or a dust collection filter, water-insoluble impurities such as tar, dust, and the like may be removed.

(S2) is a step of steam reforming methane contained in the first mixed gas in a first fixed-bed reactor to produce a second mixed gas. In (S2), a reforming reaction according to the following Reaction Formula 5 may be involved:

[Reaction Formula 5] CH₄ + H₂O → CO + 3H₂ (water vapor reforming reaction).

The reforming reaction of (S2) may be performed at a temperature of 600°C to 1400°C and a pressure of 30 KPa to 2000 KPa.

(S2) may be operated without a catalyst, but may be operated using a catalyst in order to increase a reaction conversion rate at a low temperature of 600°C to 700°C. The catalyst of (S2) may be a composite catalyst in which an active metal is supported on a support. The active metal may include one or more selected from the group consisting of nickel, vanadium, iron, platinum, palladium, and ruthenium. Usually, the active metal may be nickel, vanadium, or iron, and when an impurity content in a raw material such as organic wastes is low in the heat treatment process, precious metals such as platinum, palladium, or ruthenium may be used.

In an example, the support may be a solid acid material such as an oxide or zeolite, and specifically, may be one or more selected from the group consisting of ZSM-5, ZSM-11, USY zeolite, ferrierite, mordenite, MCM-22, SUZ-4, L-type zeolite, silica, alumina, silica-alumina, carbon, zirconia, and titania.

Since the methane steam reforming reaction of (S2) is performed in a fixed-bed reactor, the reforming reactivity of methane and the process stability may be improved to produce the synthetic gas in a high yield, thereby improving the yield of hydrocarbon. Specifically, when methane is reformed using a steam reforming reaction, reforming reactivity is improved as compared with dry reforming, an effect of removing impurities such as chlorine is exhibited, and carbon accumulation in a reforming catalyst hardly occurs, thereby performing a continuous process. In addition, since a fixed-bed reactor having the highest conversion rate per catalyst weight is used, a methane steam reforming reaction may be performed more efficiently.

(S3) is a step of separating the second mixed gas into a first stream including carbon dioxide and a second stream including hydrogen and carbon monoxide.

In an example, a method for separating the second mixed gas into a first stream and a second stream is not limited as long as it is known in the art, but in the present disclosure, the separation may be performed using a carbon dioxide separation unit, and the carbon dioxide separation unit may be an amine scrubber. Usually, the amine scrubber is binding and removing carbon dioxide through an amine-based material, and since it may separate components such as carbon dioxide and hydrogen sulfide from gas steam and recover gas containing hydrogen, carbon monoxide, or inert gas, when the amine scrubber is used, the first mixed gas may be separated into the first stream and the second stream.

As another example, the carbon dioxide separation unit may be a carbon capture and storage (CCS) unit. When the CCS unit is used for separation of carbon dioxide, it may adsorb and separate carbon dioxide using an adsorbent including any one or two or more selected from calcium oxide, calcium hydroxide, dolomite, limestone, or trona, and thus, the second mixed gas may be separated into the first and second streams using the carbon capture and storage (CCS) unit.

The first stream may include carbon dioxide at 40 vol% or more, 50 vol% or more, or 60 vol% or more as the lower limit and 99 vol% or less, 90 vol% or less, 80 vol% or less, or 70 vol% or less as the upper limit. Specifically, the first stream may include 40 to 99 vol%, more specifically 50 to 80 vol% of carbon dioxide. In the carbon dioxide separation unit, when carbon dioxide is captured and then separated, in order to separate carbon dioxide to a high purity, a regeneration tower in which carbon dioxide separation from an adsorbing material occurs should be designed with a high stage, and thus, more energy is consumed. Therefore, since the first stream includes carbon dioxide in the range described above, a carbon dioxide separation process may be performed under slightly milder conditions.

(S4) is a step of converting the first stream separated in (S3) into carbon monoxide through a reverse Boudouard reaction in a second fixed-bed reactor. Since carbon dioxide contained in the first stream is further converted into carbon monoxide through the reverse Boudouard reaction, environmental pollution may be prevented in terms of decreasing emitted carbon dioxide, while maximizing the yield of synthetic gas. The reverse Boudouard reaction may involve the following Reaction Formula 6:

[Reaction Formula 6] C+CO₂ → 2CO.

(S4) may be performed at temperature of 600°C to 1000°C and a pressure of 50 KPa 300 KPa.

The reverse Boudouard reaction may be performed by being supplied with a carbon source from outside. Specifically, the carbon source may be char derived from the organic wastes, and more specifically, may be char derived from pyrolysis of waste plastic or biomass-derived char. Otherwise, in order to prevent a possibility of impurity gases flowing in by an external carbon source, high-purity graphite may be included.

By performing the reverse Boudouard reaction of (S4) in the second fixed-bed reactor, even when the first stream includes carbon dioxide in the range described above, carbon dioxide may be converted into carbon monoxide with high efficiency. In addition, when the present disclosure uses a fixed-bed reforming reactor in (S2), it may be advantageous in terms of improving the amount of synthetic gas produced to improve reactivity of the reverse Boudouard reaction using a fixed-bed reverse Boudouard reactor having excellent reaction efficiency in (S4).

(S5) is a step of mixing the second stream separated in the carbon dioxide separation unit and carbon monoxide converted by the reverse Boudouard reaction in (S4) to produce a third mixed gas.

The third mixed gas may include hydrogen and carbon monoxide.

(S6) is a process of adjusting a ratio between carbon monoxide and hydrogen in the third mixed gas through a water gas shift reaction to produce a synthetic gas. The third mixed gas may be converted through the water gas shift reaction so as to satisfy a ratio of hydrogen : carbon monoxide appropriate for a catalytic reaction as a subsequent process. The water gas shift reaction may involve the following Chemical Formula 7:

[Chemical Formula 7] CO + H₂O → H₂ + CO₂.

The water gas shift reaction may be performed under a catalyst including Fe and Cr. The water gas shift reaction may be performed at a temperature of 100°C to 400°C, specifically 100°C to 300°C and a pressure of 20 bar to 80 bar, specifically 25 bar to 70 bar.

The synthetic gas produced by the water gas shift reaction may have a ratio of hydrogen : carbon monoxide of 1.5 to 3:1, specifically 1.9 to 2.1:1. Since the ratio of hydrogen : carbon monoxide in the synthetic gas satisfies the range described above, a catalytic reaction process which is a subsequent process may be performed well.

(S7) is a step of converting the synthetic gas produced in (S6) into a hydrocarbon oil fraction, in which the synthetic gas may be converted into an appropriate hydrocarbon oil fraction by a catalytic reaction.

The catalytic reaction is not limited as long as it may convert the synthetic gas into a hydrocarbon oil fraction, but specifically, may be a Fischer-Tropsch reaction or a methanol and olefin conversion reaction.

In an example, when the catalytic reaction of (S7) is the Fischer-Tropsch reaction, a reaction involving the following Chemical Formula 8 may be performed using the synthetic gas produced in (S6) as a raw material:

[Chemical Formula 8] nCO + 2nH₂ → CnH₂n + nH₂O.

The Fischer-Tropsch reaction may be performed under a catalyst including cobalt, nickel, or iron, may include alumina, silica, titania, or the like as a support, and may include a precious metal such as Pt, Ru, and Re as a cocatalyst.

The Fischer-Tropsch reaction may be performed at a temperature of 100°C to 500°C, specifically 200°C to 350°C and a pressure of 10 atm to 50 atm or 10 atm to 30 atm.

In an example, when the catalytic reaction of (S7) is a methanol and olefin conversion reaction, (S7) may include a reaction of converting the synthetic gas into methanol; and converting methanol into olefin.

The reaction of converting the synthetic gas into methanol may involve the following Chemical Formula 9:

[Chemical Formula 9] CO + 2H₂ → CH₃OH.

In an example, the reaction of converting the synthetic gas into methanol may be performed under a Cu-based catalyst. Specifically, the Cu-based catalyst may be a Cu-based methanol-based synthetic catalyst, and as a support of the Cu-based catalyst, one or more selected from the group consisting of SiO₂, ZrO₂, Ga₂O₃ Al₂O₃, MgO, and TiO₂ may be used. Specifically, the Cu-based methanol-based synthetic catalyst may be Cu/Zn/Al₂O₃.

The reaction of converting the synthetic gas into methanol may be performed at 400°C to 600°C, specifically 430°C to 530°C and at 0.1 MPa to 10 MPa, specifically 0.1 MPa to 5 MPa.

The reaction of converting methanol into olefin may be performed under a zeolite-based catalyst or an AlPO₄-based molecular sieve catalyst. Specifically, the zeolite-based catalyst may be ZSM-5, and the AlPO₄-based catalyst may be a silicoaluminaphosphate (SAPO) molecular sieve catalyst, specifically, one or more selected from SAPO-5, SAPO-8, SAPO-11, SAPO-16, SAPO-17, SAPO-18, SAPO-20, SAPO-31, SAPO-34, SAPO-35, SAPO-44, and SAPO-46.

The reaction of converting methanol into olefin may be performed at a temperature of 200°C to 600°C, specifically 300°C to 500°C and at a pressure of 1 bar to 10 bar, specifically 1 bar to 5 bar.

The present disclosure provides an apparatus for producing a hydrocarbon 1 including: a pyrolysis reactor 10 which heat-treats organic wastes 100 to produce a first mixed gas 110; a first fixed-bed reactor 20 which steam-reforms the first mixed gas 110 under a catalyst to produce a second mixed gas 200; a carbon dioxide separation unit 30 which separates the second mixed gas 200 into a first stream 210 including carbon dioxide and a second stream 211 including hydrogen and carbon monoxide; a second fixed-bed reactor 40 which converts the first stream 210 into carbon monoxide through a reverse Boudouard reaction; a gas mixing unit 50 which mixes the second stream 211 and carbon monoxide converted from the second fixed-bed reactor 40 to produce a third mixed gas 220; a synthetic gas production unit 60 which converts the third mixed gas 220 into a synthetic gas 230 through a water gas shift reaction; and a hydrocarbon conversion unit 70 which converts the synthetic gas 23 into a hydrocarbon through a catalytic reaction.

The apparatus for producing a hydrocarbon 1 according to the present disclosure may convert the first mixed gas 110 into a synthetic gas and a hydrocarbon, using a fixed-bed reactor performing a methane reforming reaction and a reverse Boudouard reaction, may be installed and operated economically with low installation and operating costs, and may significantly decrease carbon dioxide emissions.

In addition, it performs a process of steam reforming methane in the first fixed-bed reactor 20, thereby showing an impurity removal effect and reducing a produced amount of coke accumulated in the catalyst, and thus, the hydrocarbon production process may be stably and continuously performed.

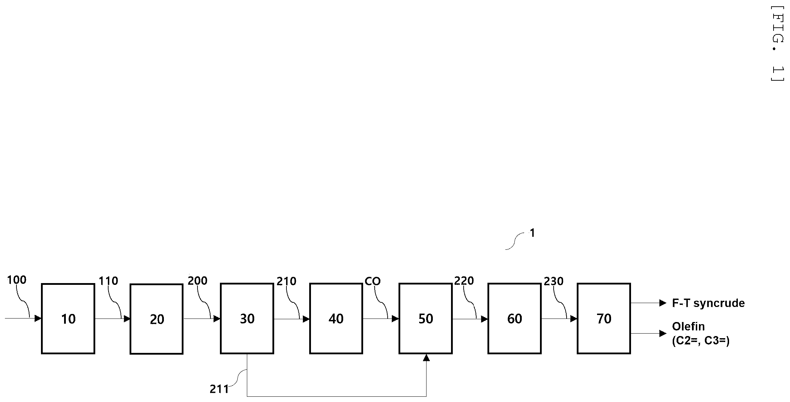

Referring to FIG. 1, the organic wastes 100 are introduced into a pyrolysis reactor 10 and heat treated to produce the first mixed gas 110. The first mixed gas 110 is introduced to the first fixed-bed reactor 20, and methane is converted into carbon monoxide and hydrogen by a steam reforming reaction to produce the second mixed gas 220.

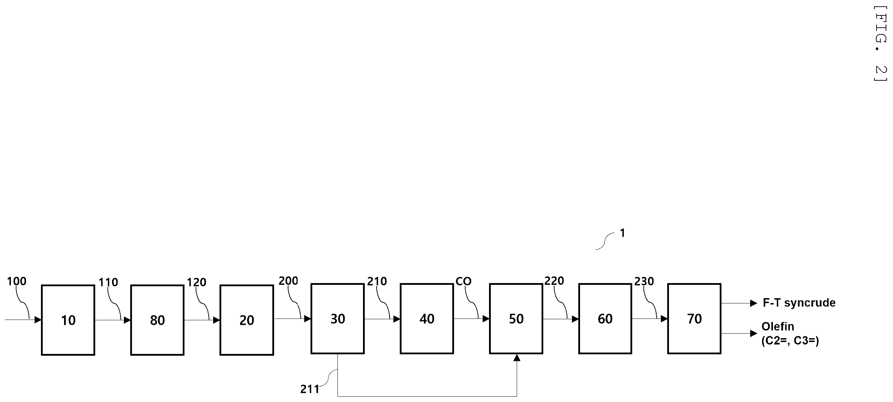

In an example, the apparatus for producing a hydrocarbon 1 may further include a purification unit 80 connected between the pyrolysis reactor 10 and the first fixed-bed reactor 20. Specifically, referring to FIG. 2, the first mixed gas 110 is introduced to the purification unit 80 to remove impurities, and the first mixed gas 120 with impurities removed is introduced to the first fixed-bed reactor 20 to perform a methane reforming reaction.

The purification unit 80 may remove water-soluble impurities such as H₂S, HCl, HOCl, and NH₃ by being supplied with the first mixed gas 110 from the pyrolysis reactor 10 by including a splinter which sprays a liquid and bringing the first mixed gas 110 and a weakly basic solution including water or sodium carbonate into contact, may remove dust using a dust collection filter for high pressure, or may remove water-insoluble impurities such as tar by including a ceramic filter.

The second mixed gas 200 is introduced to a carbon dioxide separation unit 30 and is separated into the first stream 210 including carbon dioxide and the second stream 211 including hydrogen and carbon monoxide. The second stream 211 is supplied directly to a gas mixing unit 50, and the first stream is supplied to the second fixed-bed reactor 40 and converted into carbon monoxide through the reverse Boudouard reaction.

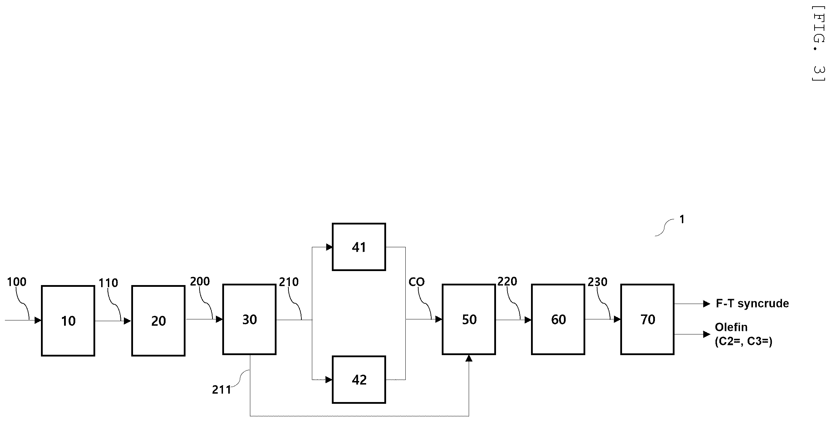

In an example, referring to FIG. 3, the second fixed-bed reactor 40 is connected between the carbon dioxide separation unit 30 and the synthetic gas production unit 60, and the second fixed-bed reactor 40 may include a fixed-bed reactor 2-1 41 and a fixed-bed reactor 2-2 42 connected in parallel. Since the second fixed-bed reactor 40 includes the fixed-bed reactor 2-1 41 and the fixed-bed reactor 2-2 42, the reactors are switched depending on the catalytic activity of the second fixed-bed reactor 40, thereby improving the entire process efficiency and allowing continuous process operation.

In another example, the second fixed-bed reactor 40 may further include a control part (not shown) which switches the fixed-bed reactor 2-1 41 and the fixed-bed reactor 2-2 42.

The control part (not shown) may control performance of a first mode in which the fixed-bed reactor 2-1 41 is regenerated, and the reverse Boudouard reaction is performed by the fixed-bed reactor 2-2 42, by blocking connection between the carbon dioxide separation unit 30 - the fixed-bed reactor 2-1 41 - the synthetic gas production unit 60.

In addition, the control part (not shown) may control performance of a second mode in which the fixed-bed reactor 2-2 42 is regenerated, and the reverse Boudouard reaction is performed by the fixed-bed reactor 2-1 41, by blocking connection between the carbon dioxide separation unit 30 - the fixed-bed reactor 2-2 42 - the synthetic gas production unit 60.

The second fixed-bed reactor 40 may be supplied with char, specifically char derived from organic wastes, and the supplied char and carbon dioxide may react to be converted into carbon monoxide. Without limitation, the char may be char derived from waste plastic pyrolysis or biomass-derived char. Otherwise, in order to prevent a possibility of impurity gases flowing in by an external carbon source, high-purity graphite may be included.

The gas mixing unit 50 mixes the second stream 211 and carbon monoxide converted in the second fixed-bed reactor 40 to produce a third mixed gas 220. The third mixed gas is supplied to the water gas shift unit 60 and converted into a synthetic gas 230 by adjusting a ratio of hydrogen : carbon monoxide in the third mixed gas 220 through a water gas shift reaction.

The synthetic gas 230 is introduced to the hydrocarbon conversion unit 70, converted into a hydrocarbon through a Fisher-Tropsch reaction or a methanol and olefin conversion reaction, and recovered into a high value-added oil fraction.

In an example, when the hydrocarbon conversion unit 70 performs a methanol and olefin conversion reaction, the hydrocarbon conversion unit 70 may include a methanol conversion unit which is supplied with the synthetic gas 230 and converts the synthetic gas into methanol and an olefin conversion unit which is supplied with methanol from the methanol conversion unit and converts methanol into an olefin.

In an example, the apparatus for producing a hydrocarbon 1 may be connected to a hydrocarbon conversion unit 70 and further include a separation step of separating the produced hydrocarbon into olefin fractions at each boiling point through distillation.

Hereinafter, the specific examples of the present disclosure will be further described.

### (Example 1)

1000 g of municipal solid wastes were added to a pyrolysis reactor, and water vapor was introduced at a temperature of 1200°C and a pressure of 250 KPa under an alumina bed and then heat treated to recover a first mixed gas having a C/O element ratio of 0.7. The composition of the recovered first mixed gas is shown in the following Table 1, and the composition of a trace amount of impurity gases included in the first mixed gas is shown in the following Table 2.

**[Table 1]**

| Feed | mol% | wt% |
|---|---|---|
| H₂ | 32 | 3 |
| CO | 16 | 18 |
| CO₂ | 40 | 72 |
| CH₄ | 11 | 7 |
| Sum | 100 | 100 |

**[Table 2]**

| Impurity Gas | ppm |
|---|---|
| HCl | 2231 |
| H₂S | 1217 |
| COS | 231 |
| NH₃ | 3701 |

The first mixed gas was cooled, added to a purification unit (wet scrubber), and treated at 70°C and 100 KPa to remove impurities, and a purified first mixed gas was recovered. As a result of treating the mixed gas through the wet scrubber, impurity gases such as NH₃, HCl, H₂S, and COS were not analyzed, and it was confirmed that impurities were effectively removed.

The first mixed gas from which impurities had been removed was supplied to a first fixed-bed reactor provided with a Ni/Al₂O₃ catalyst at a space velocity of 2 L/g_{cat}·h, thereby producing a second mixed gas through steam reforming of methane.

The Ni/Al₂O₃ catalyst was produced by impregnating an aqueous solution of nickel nitrate hexahydrate dissolved in distilled water with alumina having a diameter of 1 mm, drying at 150°C for 2 hours, and then continuously firing at 500°C for 2 hours. The process was set to a unit process, and the unit process was repeated multiple times until the nickel content in the catalyst was 20 wt%. The nickel content in the catalyst was measured through C-ray fluorescence spectrometry (XRF) analysis, and the XRF analysis was performed using ARL QUANT'X available from Thermo.

A steam reforming reaction was performed using the produced catalyst in a fixed-bed reactor. The first fixed-bed reactor was filled with 100 g of the catalyst, H₂ was supplied at a flow rate of 3.03 Nl/min at 750°C, reduction was performed for 2 hours, and the purified first mixed gas was introduced at a total flow rate of 3.33 Nl/min, and operation was performed under the conditions of a throughput of 2.0 L/g_{cat}·h, a CH₄/H₂O ratio of 3.

The second mixed gas was added to an amine scrubber to separate CO₂ from the second mixed gas, and the second stream from which carbon dioxide had been separated was recovered. Specifically, the second mixed gas was introduced to the first amine scrubber to capture CO₂ in an aqueous solution (amine solution) in which monoethanolamine (MEA) was dissolved at 50°C, and uncaptured gas was recovered as a second stream. The amine solution in the first amine scrubber was introduced to the second amine scrubber and separated into an amine solution and CO₂ at 100°C, and CO₂ was recovered as a first stream.

The recovered first stream was continuously supplied to a second fixed-bed reactor filled with a Ni/Al₂O₃ catalyst and 5 kg of high-purity graphite, and carbon dioxide was converted into carbon monoxide through the reverse Boudouard reaction. The reverse Boudouard reaction was performed in the conditions of a temperature of 750°C, a CO₂ flow rate of 2.33 Nl/min, and a space velocity of 2.0 L/g_{cat}·h under N₂ gas.

The carbon monoxide and the second converted from the first stream were introduced to the gas mixing unit and mixed at 200°C to produce a third mixed gas. The third mixed gas was introduced to a synthetic gas production unit to produce the synthetic gas having a mole ratio of H₂:CO=2:1 through a water gas shift reaction.

The synthetic gas was supplied to a hydrocarbon conversion unit, an injection speed was set so that a space velocity was 5000L/kg_{cat}·h, a volume ratio of carbon monoxide: hydrogen: argon was 63.2:31.3:5.5 under a Co/ZnO (cobalt zinc oxide)-based catalyst, and Fischer-Tropsch reaction was performed at a reaction temperature of 300°C and a pressure of 10 bar for 60 hours to recover a hydrocarbon oil fraction.

### (Comparative Example 1)

A hydrocarbon was produced and then recovered in the same manner as in Example 1, except that the reverse Boudouard reaction was not performed.

### (Comparative Example 2)

The process was performed in the same manner as in Example 1, except that the water gas shift reaction was not performed.

### (Experimental Example 1) Analysis of gas composition

When the hydrocarbon was produced by the methods of Example 1, Comparative Example 1, and Comparative Example 2, the compositions of the gases included in the first mixed gas, the second mixed gas, the third mixed gas, and the synthetic gas were analyzed and are shown in the following

Table 3, and the composition of the main components included in the hydrocarbon produced therefrom was analyzed and is shown in Table 4. Gas composition analysis was performed through gas chromatography (GC), and the total amount of gas was confirmed through a gas meter. Specifically, quantitation was performed by GC to calculate selectivity by gas, and each composition by gas was analyzed by the total amount of gas confirmed by the gas meter.

**[Table 3]**

| | Composition | Example 1 | Comparative Example 1 | Comparative Example 2 |
|---|---|---|---|---|
| First mixed gas | H₂ | 6.91 | 6.91 | 6.91 |
| | CO | 3.46 | 3.46 | 3.46 |
| (mol) | CH₄ | 2.39 | 2.39 | 2.39 |
| | CO₂ | 8.92 | 8.92 | 8.92 |
| | H₂O | 6.35 | 6.35 | 6.35 |
| Second mixed gas (mol) | H₂ | 11.23 | 11.23 | 11.23 |
| | CO | 9.05 | 9.05 | 9.05 |
| | CH₄ | 0.04 | 0.04 | 0.04 |
| | CO₂ | 5.56 | 5.56 | 5.56 |
| | H₂O | 7.11 | 7.11 | 7.11 |
| Second stream (mol) | H₂ | 11.02 | - | 11.02 |
| | CO | 8.93 | - | 8.93 |
| | CH₄ | 0.03 | - | 0.03 |
| | CO₂ | 0.02 | - | 0.02 |
| Third mixed gas (mol) | H₂ | 10.79 | - | 10.79 |
| | CO | 19.55 | - | 19.55 |
| | CH₄ | 0.01 | - | 0.01 |
| | CO₂ | 0.2 | - | 0.2 |
| Synthetic gas (mol) | H₂ | 20.12 | 11.30 | - |
| | CO | 10.01 | 5.86 | - |
| | CH₄ | 0.01 | 0.01 | - |
| | CO₂ | 0.17 | 0.27 | - |

**[Table 4]**

| | Example 1 | Comparative Example 1 | Comparative Example 2 |
|---|---|---|---|
| Gas (g) | 117.6 | 63.3 | 60.7 |
| Liquid, Wax (g) | 88.1 | 43.9 | 47.0 |
| H₂O (g) | 114.7 | 75.4 | 64.0 |

As shown in Tables 3 and 4, when a hydrocarbon was produced by the method of Example 1, carbon dioxide included in the second mixed gas was recovered through the reverse Boudouard reaction and converted into carbon monoxide, and thus, the production amount of the synthetic gas and the hydrocarbon produced therefrom was increased, and high-quality synthetic gas was obtained. In addition, the synthetic gas of Example 1 produced by performing the dry reforming reaction, the reverse Boudouard reaction, and the water gas shift process sequentially was measured to have high contents of H₂ and Co than that of CH₄ and CO₂. In addition, since the synthetic gas production yield was improved, a liquid yield of the hydrocarbon produced using the synthetic gas was as high as 88.1 g. Thus, high value-added synthetic gas and hydrocarbon may be produced from a mixed gas formed by pyrolysis or organic wastes with a high yield from the method for producing a hydrocarbon of the present disclosure, and also, since greenhouse gas emissions are reduced, an excellent environmental pollution prevention effect is shown.

However, in Comparative Example 1, since carbon dioxide included in the second mixed gas was not recovered through the reverse Boudouard reaction, the amount of the synthetic gas obtained was small as compared with Example 1, and since a large amount of carbon dioxide was included in the synthetic gas, a hydrocarbon oil liquid yield was as low as 43.9 g. In Comparative Example 2, since a water gas shift process of the third mixed gas was not performed, a mole ratio of hydrogen : carbon monoxide of the synthetic gas was not able to be controlled, and thus, since the hydrocarbon conversion reaction through the Fischer-Tropsch reaction was not performed well, the yield of the hydrocarbon liquid was measured as low as 47.0 g.

Therefore, when the hydrocarbon is produced by the method according to the present disclosure, process efficiency is improved, and thus, the synthetic gas and the hydrocarbon may be produced in a high yield. Specifically, the water vapor reforming of methane and the reverse Boudouard reaction are performed using the high fixed-bed reactor to improve methane reforming efficiency, which is good for low operating costs and easy mass production. In particular, since the reverse Boudouard reaction separates carbon dioxide included in the mixed gas and converts carbon dioxide into carbon monoxide, carbon dioxide emissions may be reduced, and also a large amount of carbon monoxide may be produced. In addition, since the synthetic gas having a controlled ratio of hydrogen and carbon monoxide included in the mixed gas is produced through the water gas shift process after the reverse Boudouard reaction, the catalytic reaction as a subsequent process is efficiently performed, and thus, the production yield of the synthetic gas and hydrocarbon may be significantly improved.

Hereinabove, although the present disclosure has been described by the specific matters and limited exemplary embodiments in the present disclosure, they have been provided only for assisting the entire understanding of the present disclosure, and the present disclosure is not limited to the exemplary embodiments, and various modifications and changes may be made by those skilled in the art to which the present disclosure pertains from the description.

Therefore, the spirit of the present disclosure should not be limited to the above-described exemplary embodiments, and the following claims as well as all modifications equal or equivalent to the claims are intended to fall within the scope and spirit of the disclosure.

### [Detailed Description of Main Elements]

| | | | |
|---|---|---|---|
| 1: | Apparatus for producing hydrocarbon | 10: | Pyrolysis reactor |
| 20: | First fixed-bed reactor | 30: | Carbon dioxide separation unit |
| 40: | Second fixed-bed reactor | 41: | Fixed-bed reactor 2-1 |
| 42: | Fixed-bed reactor 2-2 | 50: | Gas mixing unit |
| 60: | Synthetic gas production unit | 70: | Hydrocarbon conversion unit |
| 80: | Purification unit | 100: | Organic waste |
| 110: | First mixed gas | 120: | First mixed gas with impurity removed |
| 200: | Second mixed gas | 210: | First stream |
| 211: | Second stream | 220: | Third mixed gas |
| 230: | Synthetic gas | | |

## Claims

1. A method for producing a hydrocarbon, the method comprising:
(S1) heat-treating organic wastes to produce a first mixed gas;
(S2) steam-reforming the first mixed gas in a first fixed-bed reactor to produce a second mixed gas;
(S3) separating the second mixed gas into a first stream including carbon dioxide and a second stream including hydrogen and carbon monoxide;
(S4) introducing the first stream separated in (S3) into a second fixed-bed reactor and converting the first stream into carbon monoxide through a reverse Boudouard reaction;
(S5) mixing the second stream and the carbon monoxide converted in (S4) to produce a third mixed gas;
(S6) producing a synthetic gas from the third mixed gas through a water gas shift reaction; and
(S7) producing a hydrocarbon from the synthetic gas through a catalytic reaction.

2. The method for producing a hydrocarbon of claim 1, wherein the first mixed gas further includes one or more selected from the group consisting of landfill gas, shale gas, refinery exhaust gas, and biogas.

3. The method for producing a hydrocarbon of claim 1, wherein the second fixed-bed reactor is supplied with a carbon source from outside.

4. The method for producing a hydrocarbon of claim 1, wherein the first stream includes 50 vol% or more of carbon dioxide.

5. The method for producing a hydrocarbon of claim 1, wherein the pyrolysis gas has a C/O element ratio of 0.1 or less.

6. The method for producing a hydrocarbon of claim 1, wherein (S2) is performed under a composite catalyst in which an active metal is supported on a support.

7. The method for producing a hydrocarbon of claim 6, wherein the active metal includes one or more selected from the group consisting of nickel, vanadium, iron, platinum, palladium, and ruthenium.

8. The method for producing a hydrocarbon of claim 7, wherein the support includes one or more selected from the group consisting of silica, alumina, silica-alumina, carbon, zirconia, titania, zeolite, SAPO, and ALPO.

9. The method for producing a hydrocarbon of claim 1, wherein (S2) is performed at a temperature of 700°C to 1000°C.

10. The method for producing a hydrocarbon of claim 1, wherein (S4) is performed at temperature of 600°C to 1000°C and a pressure of 50 KPa 300 KPa.

11. The method for producing a hydrocarbon of claim 1, wherein the synthetic gas in (S6) includes hydrogen and carbon monoxide and a ratio between the hydrogen and the carbon monoxide satisfies 1.8:1 to 2.2:1.

12. The method for producing a hydrocarbon of claim 1, wherein the organic wastes in (S1) are one or more selected from the group consisting of waste plastic, solid waste, biomass, waste oil, waste tire, and standard plastic garbage bags.

13. The method for producing a hydrocarbon of claim 1, further comprising purifying the first mixed gas of (S1) before (S2).

14. An apparatus for producing a hydrocarbon comprising:
a pyrolysis reactor which heat-treats organic wastes to produce a first mixed gas;
a fixed-bed reforming reactor which steam-reforms the first mixed gas under a catalyst to produce a second mixed gas;
a carbon dioxide separation unit which separates the second mixed gas into a first stream including carbon dioxide and a second stream including hydrogen and carbon monoxide;
a second fixed-bed reactor which converts the first stream into carbon monoxide through a reverse Boudouard reaction;
a gas mixing unit which mixes the second stream and carbon monoxide converted from the second fixed-bed reactor to produce a third mixed gas;
a synthetic gas production unit which converts the third mixed gas into a synthetic gas through a water gas shift reaction; and
a hydrocarbon conversion unit which converts the synthetic gas into a hydrocarbon through a catalytic reaction.

15. The apparatus for producing a hydrocarbon of claim 14, wherein the second fixed-bed reactor is connected between the carbon dioxide separation unit and the gas mixing unit, and the second fixed-bed reactor includes a fixed-bed reactor 2-1 and a fixed-bed reactor 2-2 which are connected in parallel.

16. The apparatus for producing a hydrocarbon of claim 15,
wherein the second fixed-bed reactor further includes a control part which switches the fixed-bed reactor 2-1 and the fixed-bed reactor 2-2, and
the control part may control performance of a first mode in which the fixed-bed reactor 2-1 is regenerated, and the reverse Boudouard reaction is performed by the fixed-bed reactor 2-2, by blocking a connection between the carbon dioxide separation unit - the fixed-bed reactor 2-1 - the gas mixing unit; or
a second mode in which the fixed-bed reactor 2-2 is regenerated, and the reverse Boudouard reaction is performed by the fixed-bed reactor 2-1, by blocking a connection between the carbon dioxide separation unit - the fixed-bed reactor 2-2 - the gas mixing unit.

17. The apparatus for producing a hydrocarbon of claim 14, further comprising a purification unit between the pyrolysis reactor and the first fixed-bed reactor.
